(19) Europäisches Patentamt
European Patent Office
Office européen des brevets

(11) Numéro de publication: **0 167 709**
**B1**

(12) **FASCICULE DE BREVET EUROPÉEN**

(45) Date de publication du fascicule du brevet: **06.12.89**

(21) Numéro de dépôt: **85102992.6**

(22) Date de dépôt: **15.03.85**

(51) Int. Cl.⁴: **C 07 C 35/26**, C 07 C 29/136, A 61 K 7/46, C 07 C 49/633, C 07 C 45/69, C 11 D 9/44, C 11 D 3/50

(54) **1,2,3,4,4a,5,8a-Octahydro-2,2,6,8-tétraméthyl-1-naphtalénol, son utilisation en tant qu'ingrédient parfumant et procédé pour sa préparation.**

(30) Priorité: **12.07.84 CH 3376/84**

(43) Date de publication de la demande: **15.01.86 Bulletin 86/3**

(45) Mention de la délivrance du brevet: **06.12.89 Bulletin 89/49**

(84) Etats contractants désignés: **CH DE FR GB LI NL**

(56) Documents cité:
**EP-A-0 047 154**
**FR-A-2 006 177**

**BULLETIN OF THE CHEMICAL SOCIETY OF JAPAN, vol. 48, no. 10, octobre 1975, pages 2995-2996; I. NAGAKURA et al.: "Diels-Alder reaction of 2-methyl-2-cyclohexenone and substituted butadienes1)"**

(73) Titulaire: **FIRMENICH SA, 1, route des Jeunes, CH-1211 Genève 8 (CH)**

(72) Inventeur: **Tarchini, Claudio, 3, rue du Tir au Canon, CH- 1227 Carouge (CH)**

(74) Mandataire: **Salvadori, Giuseppe, Dr., c/o Firmenich S.A. Case Postale 239, CH- 1211 Genève 8 (CH)**

**EP 0 167 709 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention a trait au domaine de la parfumerie, plus particulièrement elle concerne un composé bicyclique nouveau de formule

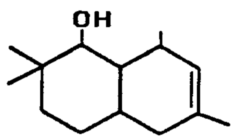

(I)

ou 1,2,3,4,4a,5,8,8a-octahydro-2,2,6,8-tétraméthyl-1-naphtalénol, composé qui possède des propriétés odorantes utiles.

L'art antérieur fait état d'une classe de composé hydroxy- et oxabicycliques définis par la formule générale que voici:

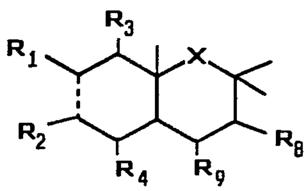

dans laquelle la ligne pointillée représente une simple ou double liaison et dans laquelle:

X représente un groupe 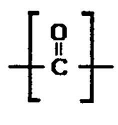 ou 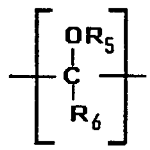

et $R_1$, $R_2$, $R_3$, $R_4$, $R_8$ et $R_9$ représentent l'hydrogène ou le groupe méthyle pour autant que (i) au moins trois des groupes $R_1$, $R_2$, $R_3$ et $R_4$ représentent un atome d'hydrogène et (ii) lorsque la ligne pointillée désigne une liaison carbone-carbone simple et X est

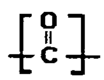 , un des $R_1$ a $R_4$ est un méthyle et les autres un hydrogène;

$R_5$ représente un hydrogène, MgZ ou Li;
Z représente chloro, bromo ou iodo; et
$R_6$ représente un hydrogène ou un méthyle.

[voir demande de brevet européen publiée sous le no. 0 047 154 le 10.3.82].

Bien que ne tombant pas sous la définition de la formule générale susindiquée, la demande citée décrit également la 1,2,3,4,4a,5,8,8a-octahydro-2,2,6,8(5,7),8a-pentaméthyl-1-naphtalénol, mélange des composés de formules

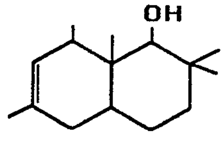 et 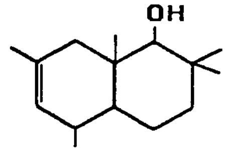

lesquels composés s'apparentent, au point de vue structurel, au composé de formule (I) de l'invention.

Malgré leur analogie de structure, le mélange de l'art antérieur et le composé de l'invention présentent des propriétés odorantes bien distinctes. En effet, le mélange décrit possède une intense odeur boisée, de type patchouli, ambrée et musquée, tandis que le composé de l'invention possède une note très montante et puissante de type fruité, rappelant les fruits d'eucalyptus ou les bourgeons de cassis. Il montre en outre un caractère hespéridé, rappelant plus particulièrement le pamplemousse ou le citron. Par certains de ses côtés, il possède également une note légèrement soufrée qui rappelle les fruits exotiques, notamment le fruit de la passion. De tels caractères olfactifs rendent les propriétés du composé (I) tout à fait surprenantes dans la mesure où la classe des composés décrits dans la demande de brevet européen citée plus haut, tout en obéissant à une structure moléculaire analogue, présente des caractères relativement uniformes de type boisé, patchouli. Par conséquent, il y avait tout lieu de penser qu'un composé homologue, tel que défini par la formule (I), possède des propriétés similaires.

De par ses propriétés, le composé de l'invention trouve un emploi avantageux dans de nombreuses applications. Il peut convenir à enrichir divers types de compositions parfumantes soient-elles hespéridées, chyprées ou fruitées par exemple.

Il est parfaitement adapté à entrer en tant que constituant tant dans des compositions alcooliques que dans des produits fonctionnels dans lesquels, grâce à sa stabilité, il trouve un emploi étendu. Il peut donc être utilisé pour le parfumage de savons, de détergents cationiques, anioniques, zwitterioniques ou non ioniques, des revitalisants textiles, des produits d'entretien, des cires ou encore des produits cosmétiques ou des shampoings. Bien entendu, le composé de l'invention peut être utilisé en tant qu'ingrédient unique, mais de préférence, il est employé en mélange avec des coingrédients parfumants usuels (à titre d'exemple on peut citer à cet effet les composés naturels ou synthétiques mentionnés dans la demande de brevet européen publiée sous le no. 0 096 243).

Les proportions dans lesquelles le composé de l'invention peut produire les effets désirés peuvent varier dans une gamme de valeurs assez étendue. De telles valeurs dépendent de l'effet particulier que l'on désire obtenir, ainsi que de la nature des autres ingrédients dans une composition donnée ou de celle des produits que l'on désire parfumer.

C'est ainsi que des concentrations de l'ordre de 0,1 - 0,5 % en poids, par rapport au poids du produit final parfumé, peuvent produire des effets marqués dans des applications telles le parfumage de savons ou de détergents. Lorsqu'il est utilisé dans des compositions parfumantes concentrées, ces proportions peuvent atteindre des valeurs bien supérieures, par exemple être de l'ordre de 2 - 5 %, voire 10 % du poids de la composition.

Comme indiqué plus haut, le composé de l'invention est une entité chimique nouvelle. Il peut être préparé conformément à un procédé analogue à celui qui a été décrit dans la demande de brevet européen no. 0 047 154, à partir de la 6,6-diméthyl-cyclohex-2-ène-1-one et du 2-méthyl-penta-1,3-diène suivant le schéma réactionnel ci-après.

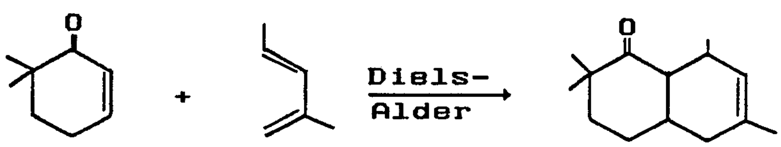


(II)

La cétone bicyclique ainsi obtenue est ensuite réduite à l'aide, par exemple, d'un alumino-hydrure d'un métal alcalin, tel l'alumino-hydrure de lithium. D'autres agents réducteurs, couramment utilisés pour effectuer la réduction du groupe carbonyle en alcool correspondant, peuvent être utilisés. C'est ainsi que l'on peut également utiliser le borhydrure de sodium, le bis (2-méthoxyéthoxy)-alumino-hydrure de sodium (VITRIDE ®, Eastman Kodak) ou le diéthylaluminohydrure de sodium (OMH-1, Ethyl Corporation). A titre de catalyseur apte à promouvoir l'addition de type Diels-Alder, il convient de mentionner le $AlCl_3$, le $TiCl_4$, le $FeCl_3$ ou le $HClO_4$. Un acide de Lewis tel le $BF_3$, de préférence en solution éthérée, est le catalyseur qui permet d'obtenir les rendements les meilleurs.

La réaction peut être effectuée soit à pression atmosphérique, soit à une pression supérieure à celle-ci. Dans ce dernier cas, l'utilisation du catalyseur n'est pas indispensable, notamment lorsqu'on opère à des températures supérieures à la température ambiante, par exemple, de l'ordre de 120 - 200°.

Le composé de l'invention, préparé conformément au procédé décrit ci-dessus, peut se présenter sous sa forme cyclanique cis (prépondérante) ou trans. La formule (I) sert donc à indiquer indifféremment les isomères suivants:

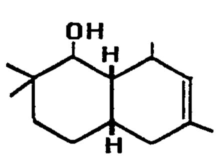

(Ia)

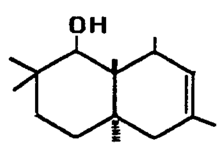

(Ib)

L'analyse spectroscopique ne nous a pas permis de mettre en évidence l'autre isomère de position possible de formule

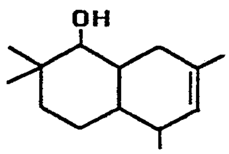

quoique sa présence en traces dans le mélange obtenu ne peut pas être exclue.

Il est en outre apparu à l'examen que le composé principal (env. 75 %) obtenu conformément au procédé décrit possède la conformation suivante

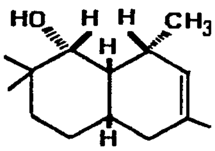

ou 1,2,3,4,4aβ,5,8,8aβ-octahydro-2,2 6,8α-tétraméthyl-1α-naphtalénol, lequel composé était accompagné de ses stéréoisomères dont la structure proposée est:

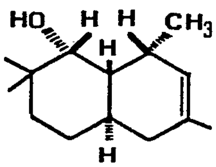

et

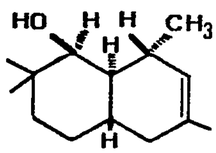

L'invention est illustrée à l'aide des exemples suivants dans lesquels les températures sont indiquées en degrés centigrades et les abréviations ont le sens usuel.

**Exemple 1**

**Préparation du 1,2,3,4,4a,5,8,8a-octahydro-2,2,6,8-tétraméthyl-1-naphtalénol**

**a. 3,4,4a,5,8,8a-Hexahydro-2,2,6,8-tétraméthyl-1(2H)-naphtalénone**

Dans un réacteur tricol de 1000 ml, muni d'un thermomètre et d'un agiteur, on a placé sous atmosphère d'azote 124 g (1 Mole) de 6,6-diméthyl-cyclohex-2-ène-1-one, 200 ml de toluène et 10 ml de trifluoro-bore éthérate. Le mélange a été refroidi à 10°C, puis on y a introduit du 2-méthyl-penta-1,3-diène par portions pendant 4 heures. Une fois l'addition terminée, le mélange de réaction a été laissé sous agitation pendant 1 heure supplémentaire, puis il a été hydrolysé avec une solution aqueuse de carbonate de sodium. L'excès de toluène a été évaporé à l'aide d'un évaporateur rotatif sous pression réduite et le résidu a été distillé pour fournir 101,7 g du produit désiré ayant Eb. 70 - 80°C/26,6 Pa. L'examen analytique nous a permis d'établir que le produit obtenu était constitué par environ 90 % d'isomère cis (a) et 10 % de trans (b). RMN(360 MHz; $CDCl_3$) : 0,99 (3H, s); 1,17 (3H, d, J = 7); 1,23 (3H, s); 1,51 (1H, d de q, J = 14 et 2,5); 1,61 (3H, large s); 2,31 (1H, m); 2,47 (1H, m); 3,03 (1H, t, J = 5); 5,23 (1H, large s) δ ppm.

**Isomère a**
SM: m/e: 206 (38), 191 (17), 173 (13), 159 (16), 135 (48), 107 (100), 91 (44).

**Isomère b**
SM: m/e: 206 (39), 191 (18), 173 (16), 159 (22), 135 (66), 107 (100), 91 (42).

**b. 1,2,3,4,4a,5,8,8a-octahydro-2,2,6,8-tétraméthyl-1-naphtalénol**

Dans un appareil tricol de 1000 ml, muni d'agiteur mécanique, d'un réfrigérant à reflux et d'un thermomètre, on a placé sous azote 5 g (132 mMole) d'alumino-hydrure de lithium et 80 ml d'éther sulfurique anhydre. Au mélange ainsi obtenue refroidi par un bain de glace, on a ajouté, pendant 1 1/2 h, 86,6 g (420 mMole) de la naphtalénone obtenue sous lettre a. ci-dessus dans 150 ml d'éther absolu. Une fois l'addition terminée, on a laissé réagir pendant 15 min. supplémentaires, puis on a hydrolysé lentement à l'aide d'acide sulfurique à 10 % jusqu'à l'obtention de deux phases séparées limpides. Après décantation, et séparation, la phase organique a été lavée jusqu'à neutralité, puis elle a été concentrée sous pression réduite. L'on a ainsi obtenu 84,1 g d'un produit brut qui, par distillation, a fourni 80 g du naphtalénol désiré ayant Eb. 90 - 100°C/26,6 Pa

RMN (360 MHz, $CDCl_3$): 0,91 (3H, s); 0,96 (3H, s); 1,11 (3H, d, J = 7); 1,66 (3H, large s); 2,02 (1H, m); 2,26 (1H, m); 2,50 (1H, m); 4,77 (1H, large s); 5,59 (1H, large s) δ ppm.

**Isomère a**
SM: m/e: 208 (3), 190 (37), 175 (48), 120 (70), 105 (100), 91 (28).

**Isomère b**
SM: m/e: 208 (1), 190 (37), 175 (37), 120 (100), 105 (93), 91 (28).

La 6,6-diméthyl-cyclohex-2-ène-1-one, utilisée comme produit de départ dans le procédé décrit ci-dessus, peut être préparée à partir d'isopropyl-méthyl-cétone et d'acroléine suivant une réaction de condensation en présence d'un agent de déshydratation acide, comme indiqué ci-dessus:

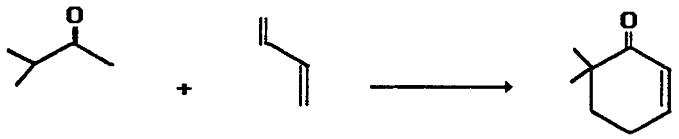

**Exemple 2**

**Parfumage de savon**

100 G de pâte de savon en copeaux, obtenus d'une base non parfumée de savon au sodium dérivée d'huile de noix de coco et de suif, ont été mélangés avec le composé préparé à l'Exemple 1, additionné à raison de 1 g. Une fois homogénéisée, la pâte de savon obtenue a servi à préparer des savonnettes, lesquelles ont été soumises à une évaluation olfactive de la part d'un groupe d'experts. Ces derniers ont établi que les savonnettes parfumées possédaient une note agréable fraîche et fruitée.

**Exemple 3**

**Eau de toilette**

On a préparé une composition parfumante de base de type Eau de Cologne masculine en mélangeant les ingrédients suivants (parties en poids):

| | |
|---|---|
| Essence de sauge sclarée | 20 |
| Essence de lavande | 150 |
| Bergamote synthétique | 200 |
| Essence de citron | 140 |
| Essence d'orange douce | 40 |
| Galbanum synthétique à 10 %* | 20 |
| Muscone à 10 %* | 50 |
| 2-Pentyl-3-oxo-cyclopentylacétate de méthyle | 10 |
| 1,1-Diméthyl-6-tert-butyl-4-acétylindane | 10 |
| α-Isométhylionone | 50 |
| Ylang synthétique | 80 |
| Jasmin synthétique | 25 |
| Géranium synthétique | 50 |
| Néroli synthétique | 100 |
| Essence de coriandre | 5 |
| Phtalate de diéthyle | 50 |
| Total | 1000 |

* dans le phtalate de diéthyle

En ajoutant à la base ci-dessus le composé obtenu à l'Exemple 1, à raison de 0,2 % en poids, on a observé un effet exaltant marqué.

**Exemple 4**

**Composition parfumante pour shampoings**

Une composition parfumante de base a été préparée en mélangeant les ingrédients suivants (parties en poids):

| | |
|---|---|
| Essence de citron déterpenée (TETRAROME ®)[1] | 300 |
| 3,5,5-Triméthylhexylacétate | 200 |
| Essence de Litsea Cubeba | 200 |
| Limonène | 50 |
| Citronellol | 40 |
| Essence de citronelle Java | 30 |
| α-Damascone à 10 % | 30 |
| Géranonitrile | 20 |
| HEDIONE ® [1] | 20 |
| Décanal à 10 %* | 20 |
| Undécanal à 10 %* | 20 |
| Dodécanal à 10 % | 20 |
| Nonanal à 10 %* | 10 |
| Formiate de citronellyle | 10 |
| RHUBOFLOR ® [1] [2] | 5 |
| Nérolidol | 5 |
| Total | 980 |

* dans le phtalate de diéthyle
1) origine: Firmenich SA, Genève
2) voir demande de brevet européen no. 66 684; 3-oxa-9- et 10-éthylidènetricyclo[6.2.1.$0^{2,7}$]undécane.

En ajoutant à la base ci-dessus 20 g du composé préparé à l'Exemple 1, on augmente le caractère naturel de la composition dont l'odeur devient plus citronnée.

**Exemple 5**

**Composition parfumante**

Une composition parfumante de base a été préparée en mélangeant les ingrédients suivants (parties en poids):

| | |
|---|---|
| Salicylate de benzyle | 150 |
| Musc cétone | 120 |
| Isométhyl-ionone[1](IRALIA ®) | 100 |
| Hydroxycitronellal[1](CYCLOSIA BASE) | 100 |
| Phénéthylol | 80 |
| Acétate de linalyle | 60 |
| Essence de jasmin synth. | 60 |
| Ylang coeur | 30 |
| Essence de bergamote** | 30 |
| Essence de clous de girofle | 30 |
| Essence de santal oriental | 30 |
| Civette synthétique à 1 %* | 30 |
| Essence de rose de mai synth. | 30 |
| Essence de mimosa absolu | 20 |
| Acétate de vétyvéryle | 15 |
| Ald. undécylénique à 10 %* | 15 |
| Isoeugénol | 15 |
| α-Terpinéol | 15 |
| Cyclopentadécanolide[1] | 10 |
| Acétate de Styrallyle | 5 |
| Musc synth.[1] | 5 |
| Ambre synth. | 5 |
| Alcool cinnamique | 5 |
| Total | 960 |

* dans le glycol dipropylénique
1) origine: Firmenich SA, Genève
** dépourvue de furocoumarines

Lorsqu'on additionne à la composition ainsi obtenue 40 g d'une solution à 10 % dans le glycol dipropylénique du composé obtenu suivant l'Exemple 1, on obtient une nouvelle composition possédant une note de tête très fraîche, montante et plus hespéridée.

**Exemple 6**

A l'aide de 1,2,3,4,4a,5,8,8a-octahydro-2,2,6,8-tétraméthyl-1-naphtalénol, on a procédé au parfumage des articles suivants à la concentration indiquée.

| | Article | Concentration % 1) | Odeur/aspect 2) |
|---|---|---|---|
| 1. | Eau de toilette | 5 | S/N |
| 2. | Crème de beauté | 0,4 | S/N |
| 3. | Shampoing | 0,5 | S/N |
| 4. | Déo-aérosol | 1,2 | S/N |
| 5. | Laque pour cheveux | 0,3 | S/N |
| 6. | Savon | 0,5 | S/N |
| 7. | Talc | 0,5 | S/N |
| 8. | Poudre détergent | 0,2 | S/N |
| 9. | Poudre à récurer chlorée | 0,2 | S/N |

1) parties en poids
2) se réfère à l'odeur et l'aspect du produit parfumé après stockage à 40°C pendant 1 mois. S = stable; N = couleur normale

**Revendications**

1. 1,2,3,4,4a,5,8,8a-Octahydro-2,2,6,8-tétraméthyl-1-naphtalénol.

2. Utilisation du composé selon la revendication 1 à titre d'ingrédient parfumant.

3. Composition parfumante ou produit parfumé résultant de l'utilisation selon la revendication 2.

4. A titre de produit parfumé selon la revendication 3, le produit résultant du parfumage d'une base de savon ou détergent.

5. Procédé pour la préparation du composé selon la revendication 1, caractérisé en ce qu'on:

a. additionne le 2-méthyl-penta-1,3-diène à la 6,6-diméthyl-cyclohex-2-ène-1-one dans les conditions d'une réaction de type Diels-Alder pour fournir la 3,4,4a,5,8,8a-hexahydro-2,2,6,8-tétraméthyl-1(2H)-naphtalénone, et
b. réduit la cétone ainsi obtenue pour fournir le 1,2,3,4,4a,5,8,8a-octahydro-2,2,6,8-tétraméthyl-1-naphtalénol à l'aide d'un agent réducteur du groupe carbonyle.

6. Procédé selon la revendication 5, caractérisé en ce qu'on utilise en tant qu'agent réducteur l'alumino-hydrure de lithium.

7. 3,4,4a,5,8,8a-Hexahydro-2,2,6,8-tétraméthyl-1(2H)-naphtalénone en tant que moyen pour la mise en oeuvre du procédé selon la revendication 5.

**Patentansprüche**

1. 1,2,3,4,4a,5,8,8a-Octahydro-2,2,6,8-tetramethyl-1-naphtalenol.

2. Verwendung der Verbindung gemäss Anspruch 1 als Parfümbestandteil.

3. Eine Parfümkomposition oder ein parfümiertes Produkt resultierend aus der Verwendung gemäss Anspruch 2.

4. Als parfumiertes Produkt gemäss Anspruch 3, das Produkt resultierend aus der Parfümierung einer Seife oder eines Waschmittels.

5. Verfahren zur Herstellung der Verbindung gemäss Anspruch 1, dadurch gekennzeichnet, dass man:

a) 2-Methyl-penta-1,3-diene an 6,6-Dimethyl-cyclohex-2-en-1-one, unter den Diels-Alder Reaktionsbedingungen, addiert um 3,4,4a,5,8,8a-Hexahydro-2,2,6,8-tetramethyl-1(2H)-naphtalenone zu erhalten, und
b) das so erhaltene Keton mittels eines Reduktionsmittels für Carbonylgruppen reduziert um 1,2,3,4,4a,5,8,8a-Octahydro-2,2,6,8-tetramethyl-1-naphtalenol zu erhalten.

6. Verfahren gemäss Anspruch 5, dadurch gekennzeichnet, dass man als Reduktionsmittel Lithiumaluminiumhydrid verwendet.

7. 3,4,4a,5,8,8a-Hexahydro-2,2,6,8-tetramethyl-1-(2H)-naphtalenone als Mittel zur Durchführung des Verfahrens gemäss Anspruch 5.

**Claims**

1. 1,2,3,4,4a,5,8,8a-Octahydro-2,2,6,8-tetramethyl-1-naphtalenol.

2. Utilization of the compound according to claim 1 as a perfuming ingredient.

3. Perfuming composition or perfumed article resulting from the utilization according to claim 2.

4. As a perfumed article according to claim 3, the article resulting from the perfuming of a soap or detergent base.

5. Process for the preparation of the compound according to claim 1, characterized in that:

a) 2-methyl-penta-1,3-diene is added to 6,6-dimethyl-cyclohex-2-en-1-one under the conditions of a Diels-Alder type reaction to yield 3,4,4a,5,8,8a-hexahydro-2,2,6,8-tetramethyl-1-(2H)-naphtalenone, and
b) the ketone thus obtained is reduced by means of a reducing agent of the carbonyl group to give 1,2,3,4,4a,5,8,8a-octahydro-2,2,6,8-tetramethyl-1-naphtalenol.

6. Process according to claim 5, characterized in that lithium aluminium hydride is used as the reducing agent.

7. 3,4,4a,5,8,8a-Hexahydro-2,2,6,8-tetramethyl-1-(2H)-naphtalenone as a means for carrying out the process according to claim 5.